# EUROPÄISCHE PATENTANMELDUNG

(11) **EP 2 671 950 A1**
(43) Veröffentlichungstag der Anmeldung: **11.12.2013**
(21) Anmeldenummer: 12170995.0
(22) Anmeldetag: 06.06.2012
(51) Int. Cl.: C12N 15/82

(54) **Expression und Sekretion von rekombinanten, vollständig assemblierten Proteinkomplexen durch Mikroalgen**

(71) Anmelder: PHILIPPS-UNIVERSITÄT MARBURG, 35037 Marburg (DE)
(72) Erfinder: Hempel, Fransziska, 61169 Friedberg (DE); Maier, Uwe, 35039 Marburg (DE)
(74) Vertreter: Stumpf, Peter

(57) **Zusammenfassung**

Die vorliegende Erfindung stellt ein Verfahren zur Expression und Sekretion von mindestens einem vollständig assemblierten und funktionellen Proteinkomplex mit medizinischem oder biotechnologischem Nutzen in Mikroalgen dar, wobei der Proteinkomplex ein Antikörper, Enzym, Hormon oder Impfstoff ist und dieser ins Medium sezerniert wird.

SEQUENCE LISTING

<110> Philipps-Universität Marburg

<120> Expression und Sekretion von rekombinanten, vollständig
      assemblierten Proteinkomplexen durch Mikroalgen

<130> TM662

<140> EP
<141> 2012-06-06

<160> 2

<170> PatentIn version 3.5

<210> 1
<211> 711
<212> DNA
<213> human

<220>
<221> Cl4mAb light chain gen
<222> (1)..(711)
<223> Synthetisches Konstrukt Cl4mAb light chain gene, cDNA Sequenzen
      der leichten Kette des humanen IgG Antikörpers CL4mAb mit
      angepasster Codon-Usage für die Expression in P. tricornutum

<400> 1
atggaaaccc cagcgcagct tctcttcctc ctgttgctct ggctcccagt ttcagatacc      60

actggagaaa ttgtgatgac gcagtctcca gccaccctct ctgtgtctcc aggggaacgc     120

gccaccctct cctgccgtgc cagtcagagt gttcgcagca acttggcctg gtaccagcag     180

aaacctggcc aggctccccg cctcctcatc tatgctgcat ccacccgcgc cactggtatt     240

ccagcccgtt tcagtggcag tgggtctggg acagagttca ctctcaccat cagcagcctg     300

caatctgaag atttcgcagt ttattactgt cagcagtata atgaatggtt tcggacgttc     360

ggccaaggga ccaaggtgga aatcaaacgc actgtggctg caccatctgt cttcatcttc     420

ccgccatctg atgagcagtt gaaatctgga actgcctctg ttgtgtgcct gctgaataac     480

ttctatcccc gcgaggccaa agtacagtgg aaggtggata acgccctcca atcgggtaac     540

tcccaggaga gtgtcacaga gcaggacagc aaggacagca cctacagcct cagcagcacc     600

ctgacgctga gcaaagcaga ctacgagaaa cacaaagtct acgcctgcga agtcacccat     660

cagggcctga gctcgcccgt cacaaagagc ttcaaccgtg gagagtgtta g              711

<210> 2
<211> 1404
<212> DNA
<213> human

<220>
<221> misc_feature
<222> (1)..(1404)
<223> Synthetisches Konstrukt Cl4mAb heavy chain gene,

cDNA Sequenzen der schweren Kette des humanen IgG Antikörpers
CL4mAb mit angepasster Codon-Usage für die Expression in P.
tricornutum

<400> 2
atggagtttg ggctcagctg ggttttcctc gttgctcttt tgcgcggtgt ccagtgtcag 60

gtgcagctgg tggagtctgg gggaggcgtg gtccagcctg ggcgctccct gcgcctctcc 120

tgtgtagcct ctggattcac cttcagtagc tatcctatga cctgggtccg ccaggctcca 180

ggcaaggggc tggagtgggt ggcaagtatt tcatatgacg gaagttataa atataaggta 240

gactccatga agggccgcct caccatctcc cgtgacaatt ccaagaacac gctgtatttg 300

gaaatgaaca gcctgacagc tgaggacacg gctgtgtatt actgtgcgcg cacagctttc 360

tttaacgcct atgacttctg gggccaggga accctggtca ccgtctcctc agcctccacc 420

aagggcccat cggtcttccc cctggcaccc tcctccaaga gcacctctgg gggcacagcg 480

gccctgggct gcctggtcaa ggactacttc cccgaaccgg tgacggtgtc gtggaactca 540

ggcgccctga ccagcggcgt gcacaccttc ccggctgtcc tccagtcctc aggactctac 600

tccctcagca gcgtggtgac cgtgccctcc agcagcttgg gcacccagac ctacatctgc 660

aacgtgaatc acaagcccag caacaccaag gtggacaaga aagttgagcc caaatcttgt 720

gacaaaactc acacatgccc accgtgccca gcacctgaac tcctgggggg accgtcagtc 780

ttcctcttcc ccccaaaacc caaggacacc ctcatgatct cccgcacccc tgaggtcaca 840

tgcgtggtgg tggacgtgag ccacgaagac cctgaggtca agttcaactg gtacgtggac 900

ggcgtggagg tgcataatgc caagacaaag ccgcgcgagg agcagtacaa cagcacgtac 960

cgtgtggtca gcgtcctcac cgtcctgcac caggactggc tgaatggcaa ggagtacaag 1020

tgcaaggtct ccaacaaagc cctcccagcc cccatcgaga aaaccatctc caaagccaaa 1080

gggcagcccc gcgaaccaca ggtgtacacc ctgcccccat cccgcgatga gctgaccaag 1140

aaccaggtca gcctgacctg cctggtcaaa ggcttctatc ccagcgacat cgccgtggag 1200

tgggagagca atgggcagcc ggagaacaac tacaagacca cgcctcccgt gctggactcc 1260

gtcggctcct tcttcctcta cagcaagctc accgtggaca agagccgctg gcagcagggg 1320

aacgtcttct catgctccgt gatgcatgag gctctgcaca accactacac gcagaagagc 1380

ctctccctgt ctccgggtaa atga 1404

## Beschreibung

Die vorliegende Erfindung betrifft ein Verfahren zur Expression von medizinisch oder biotechnologisch relevanten Proteinkomplexen in Mikroalgen, wobei die Proteinkomplexe vollständig assembliert und funktionell sind und ins Medium sezerniert werden.

### Allgemeines Gebiet und Stand der Technik

Es ist seit langem bekannt, dass Proteine, die medizinischen oder biotechnologischen Nutzen haben, wie z.B. Antikörper, Enzyme, Hormone und Impfstoffe, rekombinant durch Expressionssysteme in Bakterien, Hefen, tierischen bzw. humanen Zellkulturen hergestellt werden können.

Die Produktion von Proteinen in solchen bekannten Expressionssystemen ist, besonders wenn diese Proteine in größeren Mengen benötigt werden, mit verschiedenen Nachteilen verbunden.

Bakterielle Expressionssysteme haben den Nachteil, dass Bakterien keine eukaryoten posttranslationalen Modifikationen wie z.B. Glykosylierungen einfügen, die aber häufig für die Funktionalität des Proteins und gewünschte medizinische oder biotechnologische Anwendung entscheidend sind. An das Kulturmedium werden hohe Qualitätsanforderungen gestellt und es ist relativ teuer. Es besteht zudem die ständige Gefahr von Kontaminationen z.B. mit bakteriellen Toxinen, die steriles Arbeiten unbedingt erforderlich machen. In der Regel werden die exprimierten Proteine in sogenannten inclusion bodies angereichert und müssen in zusätzlichen aufwendigen Schritten isoliert werden.

Expressionssysteme mit tierischen oder humanen Zellkulturen oder in transgenen Tieren überwinden zwar den Nachteil der mangelnden Glykosylierung und liefern Proteine, die nahezu identisch mit den originalen Proteinen sind, aber die Verfahren sind aufwendig, störanfällig und der Einsatz von transgenen Tieren wird eher kritisch bewertet. In den Zellkulturen werden meist hochqualitative teure Kulturmedien eingesetzt und auch hier besteht die ständige Gefahr von Kontaminationen z.B. mit human-pathogenen Keimen, die steriles Arbeiten erforderlich machen. Der Einsatz von transgenen Tieren ist aus ethisch-moralischen Gründen umstritten, weiterhin können Pathogene, Viren oder Prionen übertragen werden. Eine Alternative stellt die Expression von Proteinen, die medizinischen oder biotechnologischen Nutzen haben, wie z.B. Antikörper, Enzyme, Hormone und Impfstoffe, in höheren Pflanzen dar. Selbst humane Proteine können in Pflanzen z.T. mit guter Funktionalität exprimiert und durch Modifikationen von Glykosylierungsenzymen auch bereits mit humanen Glykosylierungsmustern ausgestattet werden. Ein entscheidender Vorteil von pflanzlichen Expressionssystemen ist, dass die Energie der Sonne durch Photosynthese genutzt wird, keine teuren Kohlenstoffquellen zugegeben werden müssen und die Produktion eine neutrale CO₂ Bilanz aufweist. Die Kultivierung der Pflanzen ist im Freiland daher vergleichsweise günstig.

Nachteilig ist, dass die exprimierten Proteine erst aufwendig aus den Pflanzen isoliert und aufgereinigt werden müssen. Die Akzeptanz transgener Pflanzen im Freiland ist durch die ungewollte Verbreitung von transgenem Pollen und den damit verbundenen Gefahren bzw. Risiken sowie den Genehmigungsverfahren problematisch. Ein weiterer wichtiger Gesichtspunkt ist, dass Pflanzen, die für biotechnologische Verfahren genutzt werden, auf wertvollen Ackerflächen angebaut werden und somit mit der Nahrungsmittelproduktion konkurrieren.

### Aufgabe

Aufgabe der vorliegenden Erfindung ist es, ein einfaches und kostengünstiges Verfahren zur Expression und Sekretion von funktionellen Proteinkomplexen, die medizinischen oder biotechnologischen Nutzen haben, wie z.B. Antikörper, Enzyme, Hormone und Impfstoffe, bereitzustellen.

### Lösung der Aufgabe

Diese Aufgabe wird erfindungsgemäß durch die Ansprüche gelöst. Erfindungsgemäß erfolgt die Expression von Proteinkomplexen, die medizinischen oder biotechnologischen Nutzen haben, wie z.B. Antikörper, Enzyme, Hormone und Impfstoffe, in einem Expressionssystem mit Algen insbesondere mit Mikroalgen und einem entsprechenden erfindungsgemäßen Verfahren, bei dessen Durchführung mindestens ein vollständig assemblierter Proteinkomplex ins umgebende Medium sezerniert wird.

Dies hat den Vorteil, dass die geschlossene Reaktorproduktion sowie die vergleichsweise einfache Isolation des Proteinkomplexes aus dem Kulturmedium mit der kostengünstigen und CO₂-neutrale Produktion von pflanzlichen/phototrophen Systemen vereint wird.

Unter Mikroalgen versteht der Fachmann eukaryotische, pflanzenartige Lebewesen, die Photosynthese betreiben, jedoch nicht zu den eigentlichen Pflanzen gehören. Im engeren Sinne werden als Mikroalgen mikroskopisch kleine einzellige bis wenigzellige Lebewesen bezeichnet, z.B. die Kieselalgen oder Diatomeen (Bacillariophyta).

Diese Kieselalgen sind Hauptbestandteil des Meeresplanktons und stellen einen Großteil der marinen Biomasse dar. Als Sauerstoff produzierende (oxygene) Phototrophe erzeugen sie auch einen großen Teil des Sauerstoffs in der Erdatmosphäre.

Die Kultivierung dieser Mikroalgen erfolgt in Reaktoren, die mit Wasser und Nährsalzen sowie gegebenenfalls künstlichem Licht betrieben werden.

Bevorzugt erfolgt die Kultivierung der Mikroalgen in geschlossenen Photobioreaktoren, wie beispielsweise Röhren-Photoreaktoren, oder FPA-Reaktoren (Flachplatten-Airlift-Reaktoren). Die Reaktoren sind z.B. volldurchmischt, weisen eine geringe Schichtdicke und gezielte Strömungsführung im Reaktor auf. Über eingebaute statische Mischer kann eine verbesserte Licht- und Substratversorgung aller Algenzellen erreicht werden. Im Reaktor liegen die Mikroalgen in einer hohen Konzentration vor, was eine hohe Wirtschaftlichkeit des Produktionsprozesses bedeutet. Das FPA-Reaktorsystem nutzt auch schwächeres Licht optimal aus.

Wichtig ist bei der Auswahl des Reaktors ein hohes Oberflächen/VolumenVerhältnis, damit die Zelldichten höher sind, als im freien Gewässer gehaltene Kulturen von Mikroalgen.

Ein besonderer Vorteil des Expressionssystems mit Mikroalgen ist, dass die Produktion durch Photosynthese angetrieben wird und damit besonders kostengünstig ist, da keine teuren Kohlenstoffquellen zugeführt werden müssen. Die Produktion ist zudem CO₂-neutral und bietet im Gegensatz zu Pflanzen den Vorteil, dass viele Mikroalgen ein schnelles Wachstum aufweisen und keine wertvollen Ackerflächen benötigen. Ein weiterer positiver Gesichtspunkt dieses Expressionssystems ist der Sicherheitsaspekt. Die verwendeten Mikroalgen sind frei von Pathogenen wie Prionen oder Viren sowie frei von Endotoxinen. Das geschlossene Fermentersystem schließt eine ungewollte Freisetzung gentechnisch-veränderter Mikroalgen in die Umwelt aus und ermöglicht gleichzeitig eine Produktion unter kontrollierten cGMP Bedingungen (current Good Manufacturing Practice). Erfindungsgemäß können selbst vollständig assemblierte Proteinkomplexe nicht nur in Mikroalgen wie der Diatomee *Phaeodactylum tricornutum* exprimiert sondern auch ins Medium sekretiert werden, wodurch aufwendige Verfahren zu Zellaufschluss und Aufreinigung der Proteinkomplexe entfallen. Dies ist ein entscheidender Unterschied zu bisherigen Untersuchungen zur Expression von rekombinanten Proteinen in Mikroalgen.

Beispielhaft, aber nicht abschließend wird die Expression und Sekretion von Proteinkomplexen, die medizinischen oder biotechnologischen Nutzen haben, wie z.B. Antikörper, Enzyme, Hormone und Impfstoffe an der Mikroalge *Phaeodactylum tricornutum,* einer Diatomee, gezeigt.

Gerade die Expression von Proteinkomplexen, deren korrekte Assemblierung, also das Zusammenfügen aller Proteinuntereinheiten zu einem Proteinkomplex, essentiell ist, ist in bekannten Expressionssystemen aus dem Stand der Technik problematisch, da dort die Proteine häufig nicht funktionell exprimiert werden.

Mit dem erfindungsgemäßen Verfahren ist der Proteinkomplex nicht nur vollständig assembliert, sondern er wird von den Mikroalgen auch ins Medium sezerniert. Damit ist eine einfache Isolierung und Gewinnung von Proteinen, die medizinischen oder biotechnologischen Nutzen haben, wie z.B. Antikörper, Enzyme, Hormone und Impfstoffe möglich.

Beispielhaft aber nicht abschließend wird hier die Synthese und Sekretion eines funktionellen und vollständig assemblierten humanen IgG Antikörpers durch die Diatomee *Phaeodactylum tricornutum* gezeigt.

Erfindungsgemäß umfasst das Verfahren zur Expression und Sekretion von mindestens einem vollständig assemblierten Proteinkomplex in Mikroalgenzellen folgende Verfahrensschritte, die modular hintereinander geschaltet werden:
i) Synthese der Gene für den mindestens einen medizinisch oder biotechnologisch nützlichen Proteinkomplex
   wobei die Codon-Usage der Mikroalge berücksichtigt wird und die Sequenz für ein Signalpeptid ergänzt wird, sofern kein Sekretionssignal vorhanden ist und wobei die Zielsteuerungssequenzen, die in der Mikroalge zu einem intrazellulären Fehltargeting führen und die Sekretion verhindern, eliminiert werden
ii) Klonierung der Gene für den mindestens einen medizinisch oder biotechnologisch nützlichen Proteinkomplex aus Schritt i) in einen geeigneten Vektor mit mindestens einem induzierbaren Promotorsystem
iii) Transformation von Mikroalgenzellen mit einem Vektor nach Schritt ii)
iv) Selektion der Mikroalgenzellen aus Schritt iii) die den Vektor mit den Genen für den mindestens einen medizinisch oder biotechnologisch nützlichen Proteinkomplex aus Schritt ii) enthalten.
v) Screening der transformierten und selektierten Mikroalgenzellen aus Schritt iv) auf Syntheseeffizienz und Funktionalität des sezernierten mindestens einen Proteinkomplexes
vi) Wachstum der Mikroalgenzellen aus Schritt v) in einem Reaktor mit Medium

Anschließend wird das Medium mit dem ins Medium sezernierten mindestens einen medizinisch oder biotechnologisch nützlichen Proteinkomplexes direkt für medizinische oder biotechnologische Anwendungen eingesetzt und verwendet. Alternativ schließt sich ein weiterer Schritt an:
vii) Isolieren des mindestens einen medizinisch oder biotechnologisch nützlichen Proteinkomplexes aus Schritt vi) aus dem Medium

Insbesondere sind die Zielsteuerungssequenzen, die in Schritt i) eliminiert werden Sequenzen, die in der Mikroalge zu einem intrazellulären Fehltargeting führen und die Sekretion verhindern, z.B. ER-Retensionssignale, mitochondrielle Zielsteuerungssequenzen, plastidäre Zielsteuerungssequenzen, peroxisomale Zielsteuerungssequenzen, lysosomale Zielsteuerungssequenzen oder kernspezifische Zielsteuerungssequenzen.

Insbesondere erfolgt in
Schritt ii) die Klonierung der Gene in Form von einzelnen Untereinheiten des mindestens einen medizinisch oder biotechnologisch nützlichen Proteinkomplexes. Ein geeigneter Vektor mit mindestens einem induzierbaren Promotorsystem ist z.B. der Nitratreduktasepromotor/-terminator, wodurch die Proteinexpression durch Zugabe von nitrathaltigem Medium induziert werden kann.

Beispielhaft erfolgt das Screening der Transformanden auf Syntheseeffizienz und Funktionalität des sezernierten Proteinkomplexes mit einem ELISA oder kommerziellen Easy Titer Test.

Erfindungsgemäß werden für das Verfahren Mikroalgen, z.B. die Diatomee *Phaeodactylum tricornutum* verwendet. Dier Kultivierungsmethoden und das Wachstum der Mikroalgen in einem Reaktor mit geeignetem Medium sind dem Fachmann bekannt.

In einem Ausführungsbeispiel ist der mindestens eine medizinisch oder biotechnologisch nützliche Proteinkomplex ein Antikörper. In diesem Fall wird das Medium nach Schritt vi), dass den Antikörper enthält, direkt für diagnostische Anwendungen wie ELISA Analysen eingesetzt.

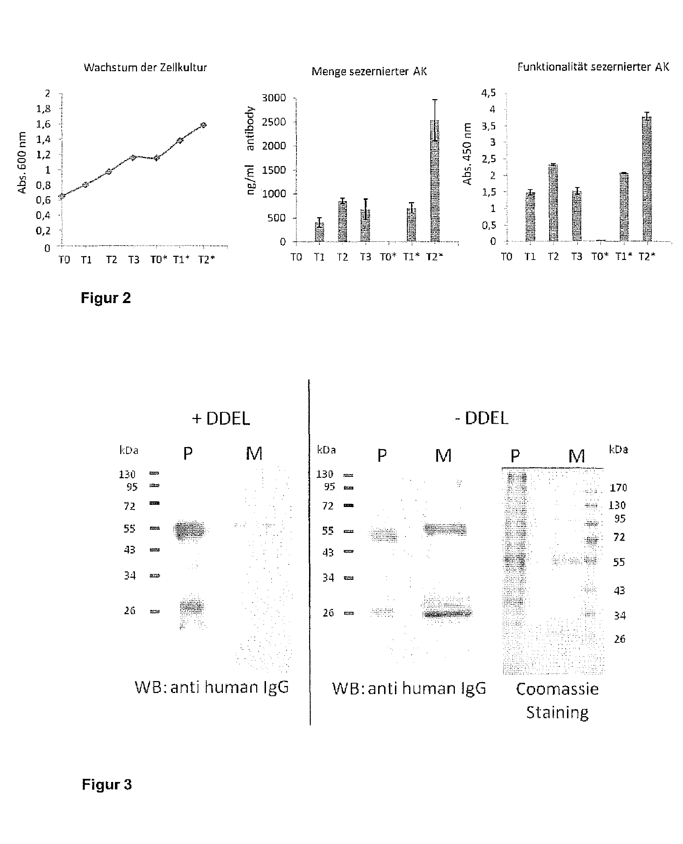

Die Sekretionsleistung des Antikörpers in das Medium liegt zwischen 500 und 3000 ng/ml je nach Dichte der Kultur und Dauer der Expression. Figur 2 zeigt beispielhaft die Sekretionsleistung bei 2500 ng/ml (Fig. 2).

Für die Sekretion des Antikörpers sind keine speziellen Modifikationen des *Phaeodactylum tricornutum* Stamms - wie die Reduktion der Zellwand oder ähnliches - notwendig.

### Ausführungsbeispiel

Im vorliegenden Beispiel wird mit dem erfindungsgemäßen Verfahren ein humaner Antikörper, z.B. der humane IgG1 Antikörper CL4mAb, der das Oberflächenprotein HBsAg des Hepatitis B Virus erkennt, hergestellt. Der Antikörper wird effizient ins Medium sekretiert und ist vollständig assembliert und biologisch funktionsfähig.

Dazu wird der humane IgG1 Antikörper CL4mAb, der das Oberflächenprotein HBsAg des Hepatitis B Virus erkennt, zunächst im endoplasmatischen Retikulum der Diatomee *Phaeodactylum tricornutum* exprimiert. Dieser Antikörper stammt ursprünglich aus der humanen Zell-Linie TAPC301-CL4 und wurde bereits zuvor im heterologen System Tabak bzw. als Fab-Fragment auch in *E. coli* exprimiert, aufgereinigt und erfolgreich getestet. Durch Deletion des C-terminalen ER-Retensionssignals, wird eine effiziente Sekretion des Antikörpers ins umgebende Medium der Mikroalgen ermöglicht.

Die vollständigen Sequenzen für die schwere und leichte Kette (also inklusive Fc-Teil) des Antikörpers IgG1/kappa Antikörper CL4mAb gegen das Hepatits B Virus Oberfiächenprotein werden in *P. tricornutum* eingebracht (Aminosäuresequenz AAB86466.2, AAB86467.2). Die Verfahren entsprechen den üblichen Klonierungsmethoden und sind dem Fachmann bekannt. Die Nukleinsäure-Sequenzen werden zuvor der Codon-Usage von *P. tricornutum* angepasst und von der Firma MrGene (Regensburg) generiert (Genbank: JF970211, JF97021 0).

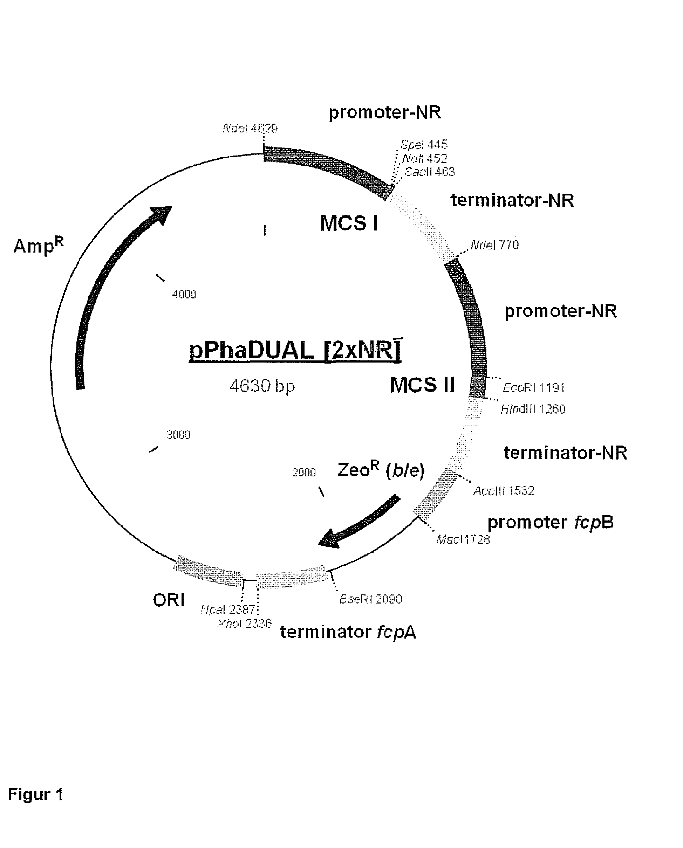

Die C-terminale Sequenz DDEL, das sogenannte ER-Retensionssignal wird entfernt, damit die Proteine in das Medium sezerniert werden können und sich nicht in der Mikroalge anreichern. Die humane Zielsteuerungssequenz am N-Terminus des Antikörpers ist notwendig, um den Proteinkomplex ins Medium zu dirigieren. Dies führt im vorliegenden Ausführungsbeispiel dazu, dass der Antikörper nun effizient mit bis zu 500 bis 3000 ng/ml ins Medium abgegeben wird (Fig. 1 und Fig. 2).

Für die effiziente Sekretion ist kein spezieller Stamm an Mikroalgen mit reduzierter Zellwand oder ähnlichem notwendig.

Zur Klonierung wird beispielsweise der Vektor pPha-DUAL[2xNR] verwendet (GenBank: JN180664). Dies ist ein Plasmid, das sich von dem Vektor pPhaT1 (GenBank: AF219942) ableitet. Das Plasmid pPhaN-DUAL[2xNR] zeichnet sich dadurch aus, dass es eine weitere MCS (Multiple cloning site) besitzt und beide MCSs unter dem induzierbaren Promotor/Terminator der Nitratreduktase von *P. tricornutum* stehen Figur 3 zeigt die Vektorkarte. Das Expressionsystem kann durch Zugabe von Nitrat induziert werden, so dass eine optimale Expressionszeit ermittelt und diese reguliert werden kann. Die beste Sekretionseffizienz mit 2500 ng/ml wird bei hohen Zelldichten erreicht (OD 600nm ≥ 1) und einer Induktionszeit von etwa 2 Tagen (Fig. 2). Ein Mediumwechsel nach 3 Tagen zeigt, dass dies die Synthese/Sekretionsleistung noch mal bis auf 3000 ng/ml erhöht und auch eine kontinuierliche Kultivierung der Zellen möglich ist - ein wichtiger Faktor für die Umsetzung einer kostengünstigen Kultivierung und Produktion im großem Maßstab (Fig. 2). Grundsätzlich ist auch eine dauerhafte Induktion in NO₃-Medium möglich, da das Zellwachstum durch die Antikörperproduktion nicht beeinträchtigt wird, allerdings liefert eine Dauerinduktion geringere Ausbeuten mit Höchstwerten von ca. 550 ng/ml Antikörper im Medium.

Die Transformation der Mikroalge *P. tricornutum* erfolgt mittels der dem Fachmann bekannten Methode des Partikelbombardements. Dabei werden M10 Wolframpartikel und 1350 psi Rupture disks für die Transfektion mit dem Bio-Rad Biolistic PDS-1000/He verwendet.

Die Selektion erfolgt mit dem Antibiotikum Zeocin in einer Konzentration von 75 µg/ml.

Die Mikroalge *Phaeodactylum tricornutum* (Bohlin, University of Texas Culture Collection, strain 64) wird in f/2 Medium unter Standardbedingungen mit entweder 0,9 mM NO₃ oder 1,5 mM NH₄ als Stickstoffquelle kultiviert. Die Zellen werden in 150 ml Ansätzen bei 22°C und konstanter Illumination (80 mmol photons per m² per s) unter Schütteln (150 rpm) angezogen.

Zur Analyse der Menge an sezerniertem Antikörper werden die Zellen pelletiert (10 min, 1500 x g) und das Medium filtriert (Porendurchmesser 0,2 µm), um sicherzustellen, dass keine intakten Zellen mehr im Überstand enthalten sind. Zur Bestimmung der Antikörpermenge wird beispielsweise das Easy Titer Kit Easy-Titer human IgG (H+L) Assay Kit (ThermoScientific) verwendet. Die Funktionalitätsanalysen wurden mit einem ELISA-Test (z.B. einem ELISA-Test nach Hempel et al. 2011 a) beschrieben. Abhängig von der Dichte der Kultur variiert die Menge an funktionellem Antikörper im Medium und erreicht 500ng/ml bis 3000 ng/ml.

Das filtrierte Medium mit dem sezernierten Antikörper wird direkt für die Quantifizierung sowie ELISA Analysen eingesetzt.

Weitere Aufreinigungsschritte des Proteins sind nicht zwangsläufig notwendig. Figur 1 zeigt in der Coomassie-Färbung eines Proteingels, dass das Medium neben dem sezernierten Antikörper keine weiteren Proteine in größeren Mengen enthält. Das Medium kann zu diagnostischen Zwecken wie ELISA folglich direkt verwendet werden oder aber der Antikörper wird alternativ zuvor z.B. durch ProteinA Sepharose nach Herstellerangaben aufgereinigt.

Weiterhin werden alternativ Schritte zur Ankonzentrierung des sezernierten Antikörpers oder der Austausch des Mediums gegen einen adäquaten Puffer zur Langzeitlagerung angeschlossen.

### Abbildungslegende

**Fig. 1** zeigt Vektorkarte zu pPhaDUAL [2xNR]. Beide Multiple Cloning Sites (MCS I und MCSII) stehen unter dem Nitratreduktase (NR) Promotor von *P.tricornutum.*
**Fig. 2** zeigt die schematische Auswertung von Untersuchungen zu Quantität und Funktion des sezernierten Antikörpers in Relation zum Wachstum der Kultur. Die Expression und Sekretion des Antikörpers wird bei einer optischen Dichte von ca. 0,6 eingeleitet (T0). Über einen Zeitraum von 3 Tagen wurden Proben des Mediums genommen, filtriert und die Menge sowie die Funktionalität des sezernierten Antikörpers ermittelt (T1, T2, T3). Nach 3 Tagen wird das Medium mit den darin enthaltenen Antikörpern komplett entfernt und durch frisches Nitrat-haltiges Medium ersetzt (T0*) und Proben über 2 weitere Tage entnommen. Abs. - Absorption, AK - Antikörper
**Fig. 3** zeigt eine Analyse des sekretierten humanen IgG Antikörpers Cl4mAb aus *P. tricornutum.* Die Western Blot Analysen zeigen, dass bei einer Expression des Antikörper mit einem ER-Retensionssignal (+DDEL) nur ein kleiner Teil ins Medium abgegeben wird (M) und der Großteil im Zellpellet verbleibt. Durch Deletion des ER-Retensionssignals (-DDEL) wird der Antikörper effizient ins Medium abgegeben. Eine vergleichende Coomassie-Färbung des Proteingels (links) macht deutlich, dass neben dem sezernierten Antikörper kaum weitere Proteine im Medium vorhanden sind. Für die Western Blot Analysen werden 15 ml Medium einer dichten Kultur filtriert, ankonzentriert, gefällt und gelelektrophoretisch aufgetrennt (M). Zur Kontrolle werden 10 µg Gesamtprotein des Zellpellets aufgetragen (P). Für die Coomassie-Färbung werden 30 ml Medium filtriert, ankonzentriert und gefällt.

## Patentansprüche

1. Verfahren zur Expression und Sekretion von mindestens einem vollständig assemblierten Proteinkomplex in Mikroalgenzellen umfassend die Schritte:
i) Synthese der Gene für den mindestens einen medizinisch oder biotechnologisch nützlichen Proteinkomplex
wobei die Codon-Usage der Mikroalge berücksichtigt wird und die Sequenz für ein Signalpeptid ergänzt wird, sofern kein Sekretionssignal vorhanden ist und wobei die Zielsteuerungssequenzen, die in der Mikroalge zu einem intrazellulären Fehltargeting führen und die Sekretion verhindern, eliminiert werden
ii) Klonierung der Gene für den mindestens einen medizinisch oder biotechnologisch nützlichen Proteinkomplex aus Schritt i) in einen geeigneten Vektor mit mindestens einem induzierbaren Promotorsystem
iii) Transformation von Mikroalgenzellen mit einem Vektor nach Schritt ii)
iv) Selektion der Mikroalgenzellen aus Schritt iii), die den Vektor mit den Genen für den mindestens einen medizinisch oder biotechnologisch nützlichen Proteinkomplex aus Schritt ii) enthalten
v) Screening der transformierten und selektierten Mikroalgenzellen aus Schritt iv) auf Syntheseeffzienz und Funktionalität des sezernierten mindestens einen Proteinkomplexes
vi) Wachstum der Mikroalgenzellen aus Schritt v) in einem Reaktor mit Medium

2. Verfahren zur Expression und Sekretion von mindestens einem vollständig assemblierten Proteinkomplex in Mikroalgenzellen nach Anspruch 1, **dadurch gekennzeichnet, dass** der mindestens eine medizinisch oder biotechnologisch nützliche Proteinkomplex nach Schritt vi) aus dem Medium isoliert wird.

3. Verfahren zur Expression und Sekretion von mindestens einem vollständig assemblierten Proteinkomplex in Mikroalgenzellen nach Anspruch 1 und 2, **dadurch gekennzeichnet, dass** der mindestens eine medizinisch oder biotechnologisch nützliche Proteinkomplex ein Antikörper, Enzym, Hormon oder Impfstoff ist.

4. Verfahren zur Expression und Sekretion von mindestens einem vollständig assemblierten Proteinkomplex in Mikroalgenzellen nach Anspruch 3, **dadurch gekennzeichnet, dass** der mindestens eine medizinisch oder biotechnologisch nützliche Proteinkomplex ein humaner IgG Antikörper ist.

5. Verfahren zur Expression und Sekretion von mindestens einem vollständig assemblierten Proteinkomplex in Mikroalgenzellen gemäß einem der vorangegangenen Ansprüche, **dadurch gekennzeichnet, dass** die Mikroalge eine Diatomee ist.

6. Verfahren zur Expression und Sekretion von mindestens einem vollständig assemblierten Proteinkomplex in Mikroalgenzellen gemäß Anspruch 5, **dadurch gekennzeichnet, dass** die Mikroalge *Phaeodactylum tricornutum* ist.

7. Vollständig assemblierter Proteinkomplex hergestellt mit einem Verfahren gemäß der Ansprüche 1 bis 6 in Mikroalgenzellen.

8. Verwendung eines vollständig assemblierten Proteinkomplexes gemäß gemäß Anspruch 7 für medizinische oder biotechnologische Anwendungen.

9. Mikroalgenzelle deren Zielsteuerungssequenzen, die zu einem intrazellulären Fehltargeting führen und die Sekretion verhindern, eliminiert wurden umfassend Sequenzen für ein Signalpeptid und einen Vektor nach Schritt ii), mit Genen, die für mindestens einen medizinisch oder biotechnologisch nützlichen Proteinkomplex kodieren.

10. Mikroalgenzelle gemäß Anspruch 9, **dadurch gekennzeichnet dass**, der mindestens eine medizinisch oder biotechnologisch nützliche Proteinkomplex ein Antikörper, Enzym, Hormon oder Impfstoff ist.

11. Mikroalgenzelle gemäß Anspruch 10, **dadurch gekennzeichnet dass**, der mindestens eine medizinisch oder biotechnologisch nützliche Proteinkomplex ein humaner IgG Antikörper ist.

12. Mikroalgenzelle gemäß Anspruch 9 bis 11, **dadurch gekennzeichnet dass**, sie der Gattung *Phaeodactylum tricornutum* angehört.
